# EUROPEAN PATENT SPECIFICATION

(11) **EP 3 521 449 B1**
(45) Date of publication and mention of the grant of the patent: **14.07.2021**
(21) Application number: 17856569.3
(22) Date of filing: 17.08.2017
(51) Int. Cl.: C12Q 1/68, G01N 33/68, G01N 33/577, C12Q 1/6883

(54) **BIOMARKER COMPOSITION FOR DIAGNOSIS OF SKIN BARRIER FUNCTION**
BIOMARKERZUSAMMENSETZUNG ZUR DIAGNOSE DER HAUTBARRIEREFUNKTION
COMPOSITION DE BIOMARQUEUR POUR LE DIAGNOSTIC DE LA FONCTION BARRIÈRE CUTANÉE

(30) Priority: 28.09.2016 KR 20160124663
(43) Date of publication of application: 07.08.2019
(73) Proprietor: Amorepacific Corporation, Seoul 04386 (KR)
(72) Inventor: SON, Eui Dong, Yongin-si Gyeonggi-do 17074 (KR); JEON, Byeong Bae, Yongin-si Gyeonggi-do 17074 (KR); KIM, Ji Hyun, Yongin-si Gyeonggi-do 17074 (KR); KIM, Kyu Han, Yongin-si Gyeonggi-do 17074 (KR); PARK, Sun Young, Yongin-si Gyeonggi-do 17074 (KR); KIM, Hyoung June, Yongin-si Gyeonggi-do 17074 (KR); CHOI, Min Sik, Yongin-si Gyeonggi-do 17074 (KR); CHO, Eun-Gyung, Yongin-si Gyeonggi-do 17074 (KR); LEE, Tae Ryong, Yongin-si Gyeonggi-do 17074 (KR)
(74) Representative: Novagraaf Technologies
(86) International application number: PCT/KR2017/008955
(87) International publication number: WO 2018/062683

(56) References cited:
- JP-A- 2016 037 470
- KR-A- 20140 089 505
- US-A1- 2010 255 478
- US-A1- 2011 212 455
- US-A1- 2013 236 904
- DANIELA BUSSE ET AL: "A Synthetic Sandalwood Odorant Induces Wound-Healing Processes in Human Keratinocytes via the Olfactory Receptor OR2AT4", JOURNAL OF INVESTIGATIVE DERMATOLOGY, vol. 134, no. 11, 1 November 2014 (2014-11-01), pages 2823-2832, XP055534614, NL ISSN: 0022-202X, DOI: 10.1038/jid.2014.273 & DANIELA BUSSE ET AL: "A Synthetic Sandalwood Odorant Induces Wound-Healing Processes in Human Keratinocytes via the Olfactory Receptor OR2AT4 Supplemental information - Materials & Methods:", JOURNAL OF INVESTIGATIVE DERMATOLOGY, vol. 134, 7 August 2014 (2014-08-07), pages 1-15, XP055679556,
- Kamya Biomedical Company: "Olfactory Receptor 6M1 (OR6V1) Elisa Kit, Human", , 26 April 2016 (2016-04-26), XP055679544, Retrieved from the Internet: URL:http://www.kamiyabiomedical.com/cgi-bi n/search.cgi?search=or6v1&table=assaykits& species=&int=&app=&language=english [retrieved on 2020-03-25]
- Kamya Biomedical Company: "Olfactory Receptor 6M1 (OR6M1) Elisa Kit, Human", , 26 April 2016 (2016-04-26), XP055679540, Retrieved from the Internet: URL:http://www.kamiyabiomedical.com/cgi-bi n/search.cgi?search=or6m1&table=assaykits& species=&int=&app=&language=english [retrieved on 2020-03-25] & Kamya: "Immunoassay reagents for Chemistry Analyzers - Kamya", , 26 April 2016 (2016-04-26), XP055679537, Retrieved from the Internet: URL:https://www.google.com/search?rlz=1C1G CEB_enDE877DE877&tbs=cdr:1,cd_max:09-28-20 16&ei=MTV7Xs-LJLDgkgXO4q_QAg&q=or6m1+catal og&oq=or6m1+catalog&gs_l=psy-ab.3..33i160. 2215.2215..2545...0.0..0.106.271.2j1...... 0....1..gws-wiz.VcEYcezMUeI&ved=0ahUKEwiP6 K-DuLXoAhUwsKQKHU7xCyoQ4dUDCAs&uact=5 [retrieved on 2020-03-25]
- BUSSE, DANIELA et al.: "A Synthetic Sandalwood Odorant Induces Wound-healing Processes in Human Keratinocytes via the Olfactory Receptor OR2 AT 4", Journal of Investigative Dennatology, vol. 134 7 August 2014 (2014-08-07), pages 2823-2832, XP055534614, DOI: doi:10.1038/jid.2014.273
- DENDA, MITSUHIRO: "Newly Discovered Olfactory Receptors in Epidermal Keratinocytes Are Associated with Proliferation, Migration, and Re-epitheliazation of Keratinocytes", Journal of Investigative Dermatology, vol. 134, no. 11, 2014, pages 2677-2679, XP055584109,
- ABAFFY, TATJANA: "Human Olfactory Receptors Expression and Their Role in Non-olfactory Tissues-a mini-review", Journal of Pharmacogenomics & Pharmacoproteomics, vol. 6, no. 4, 6 October 2015 (2015-10-06) , pages 1-7, XP055584111,

## Description

### [Technical Field]

The present specification describes a composition for diagnosis of skin barrier function, a kit for diagnosis of skin barrier function, and a method for screening a skin barrier function improving substance.

### [Background Art]

The stratum corneum, which is the outermost layer of the epidermis, functions to prevent the skin barrier from being damaged by external factors such as external humidity, temperature change, ultraviolet light and microorganisms and acts as a barrier to prevent water from evaporating out of the skin. The skin barrier is subdivided into the barrier of the stratum corneum and the tight junction which prevents water evaporation through intercellular adhesion below the stratum corneum. It is known that a decrease in skin moisture in patients with atopic dermatitis is related to a decrease in intercellular lipid of the stratum corneum due to abnormal epidermal differentiation and the weakening of tight junction due to a decrease in claudin (J Allergy Clin Immunol. 2011 Mar; 127 (3): 773-86). Hence, the evaluation on abnormal epidermal differentiation or tight junction is a representative method for evaluating skin barrier damage.

Recently, it has been known that the olfactory receptors present in the nose are also present in various organs and skin. It is known that the function of the olfactory receptors present in the organs or skin unlike the nose is different from that in the nose, but the specific functions of the various olfactory receptors the functions of which in the sites other than the nose are not all known, and it is expected that the respective receptors have different specific functions.

### [Summary of Invention]

### [Technical Problem]

In an aspect, a problem to be solved by the present description is to provide a unit for objective diagnosis of skin barrier function.

In another aspect, a problem to be solved by the present description is to provide a unit for objective evaluation on the promotion of epidermal cell differentiation.

In still another aspect, a problem to be solved by the present description is to provide a unit for objective evaluation on the enhancement of skin moisturization.

In still another aspect, a problem to be solved by the present description is to provide a unit for objective evaluation on the improvement of tight junction.

In still another aspect, a problem to be solved by the present description is to provide a method for screening a skin barrier improving substance.

In still another aspect, a problem to be solved by the present description is to provide a method for screening an epidermal cell differentiating substance.

In still another aspect, a problem to be solved by the present description is to provide a method for screening a skin moisturizing substance.

In still another aspect, a problem to be solved by the present description is to provide a method for screening a tight junction improving substance.

### [Solution to Problem]

In an aspect, disclosed therein is a method for diagnosing the skin barrier function, comprising testing the expression level of at least one gene of OR6M1 and OR6V1 in a specimen.

In another aspect, disclosed therein is a composition for diagnosis of skin barrier function, with which the expression level of at least one gene of OR6M1 and OR6V1 in a specimen is tested.

In still another aspect, disclosed therein is a kit for diagnosis of skin barrier function, with which the expression level of at least one gene of OR6M1 and OR6V1 in a specimen is tested.

In still another aspect, disclosed therein is an ex vivo method for screening a skin barrier function improving substance, comprising treating an epidermal cell with a test substance; and confirming relative expression levels of at least one gene of OR6M1 and OR6V1 before and after the treatment with a test substance in the epidermal cell treated with a test substance.

### [Advantageous Effects of Invention]

In an aspect, the present description can provide a unit for objective diagnosis of skin barrier function.

In another aspect, the present description can provide a unit for objective evaluation on the promotion of epidermal cell differentiation.

In still another aspect, the present description can provide a unit for objective evaluation on the enhancement of skin moisturization.

In still another aspect, the present description can provide a unit for objective evaluation on the improvement of tight junction.

In still another aspect, the present description can provide a method for screening a skin barrier improving substance.

In still another aspect, the present description can provide a method for screening an epidermal cell differentiating substance.

In still another aspect, the present description can provide a method for screening a skin moisturizing substance.

In still another aspect, the present description can provide a method for screening a tight junction improving substance.

The invention is defined in the appended claims.

### [Brief Description of Drawings]

FIG. 1 illustrates the expression levels of OR6M1 gene and OR6V1 gene in keratinocytes depending on the number of days elapsed (day 2, day 4, and day 6).
FIG. 2 illustrates the expression levels of OR6M1 gene and OR6V1 gene in keratinocytes after being irradiated with ultraviolet ray (UVB: 25 mJ/cm²).
FIG. 3 is a graph illustrating the results of knock-down of OR6M1 gene and OR6V1 gene, respectively.
FIG. 4 illustrates changes in the expression levels of KRT1, KRT10, and LOR, which are genes related to epidermal differentiation, when OR6M1 gene and OR6V1 gene are knocked down.
FIG. 5 illustrates changes in the expression levels of BH, FLG and, Caspase14, which are genes related to moisturization, when OR6M1 gene and OR6V1 gene are knocked down.
FIG. 6 illustrates changes in the expression levels of Occludin, Claudin1, and TJP1, which are genes related to tight junction, when OR6M1 gene and OR6V1 gene are knocked down.

### [Description of Embodiments]

Hereinafter, embodiments of the present description will be described in more detail. However, the techniques disclosed in the present application are not limited to the embodiments described herein but may be embodied in other forms. It should be understood, however, that the embodiments disclosed herein are provided so that this disclosure will be thorough and complete and will fully convey the scope of the invention to those skilled in the art. It will be apparent to those skilled in the art that various modifications and variations can be made in the present invention without departing from the scope of the invention.

In the present specification, the term "probe" means a polynucleotide having a base sequence which can complementarily bind to a target site of a gene, a variant thereof, or one including the polynucleotide and a labeling substance bound thereto.

In the present specification, the term "primer" means a polynucleotide having a base sequence which can complementarily bind to the end of a specific region of a gene used for amplifying a specific region corresponding to a target region of the gene using PCR or a variant thereof. The primer is not required to be completely complementary to the end of a specific region, and one that is complementary enough to hybridize with the end to form a double-stranded structure can be used.

In the present specification, the term "hybridization" means that two single-stranded nucleic acids form a duplex structure by pairing of complementary base sequences. Hybridization can occur not only in perfect complementarity between single-stranded nucleic acid sequences but also in the presence of some mismatching bases.

In the present specification, the term "polynucleotide" means a polymer of a plurality of nucleotides and means a polynucleotide in a broad sense including oligonucleotides which are polymers of tens of nucleotides in the conventional sense.

In the present specification, the term "normal value" means the expression level in a state in which undamaged skin is normally differentiated.

In an embodiment, the present description relates to a method for diagnosing the skin barrier function comprising testing the expression level of at least one gene of OR6M1 and OR6V1.

The human OR6M1 (olfactory receptor family 6 subfamily M member 1) gene encodes the olfactory receptor 6M1 protein of olfactory cells, and the NCBI accession ID of the OF6M1 gene is NM_001005325.1. The present inventors have found out that the gene plays a role of improving the skin barrier function when being expressed in epidermal cells. Hence, the OR6M1 gene can be used as a biomarker for the diagnosis of and evaluation on the skin barrier function in epidermal cells, and objective diagnosis of and evaluation on the skin barrier function is possible through the expression level test.

The human OR6V1 (olfactory receptor family 6 subfamily V member 1) gene encodes the olfactory receptor 6V1 protein of olfactory cells, and the NCBI accession ID of the OR6V1 gene is NM_001001667.1. The present inventors have found out that this gene plays a role of improving the skin barrier function when being expressed in epidermal cells. Hence, the OR6M1 gene can be used as a biomarker for the diagnosis of and evaluation on the skin barrier function in epidermal cells, and objective diagnosis of and evaluation on the skin barrier function is possible through the expression level test.

In an embodiment, the method for diagnosing the skin barrier function may comprise testing the expression level of at least one gene of OR6M1 and OR6V1 in a specimen.

The specimen may be a human epidermal cell, for example, at least one of keratinocytes or melanocyte.

When the expression level of OR6M1 or OR6V1 gene in a specimen is high, the skin barrier function is excellent so that the epidermal cell differentiation is promoted, the skin moisturization is enhanced, the intercellular tight junction of the granular layer and stratum spinosum below the stratum corneum is improved, the epidermal cells tightly bind to each other, thus the epidermal cell tissue can be solidified, and as a result, the evaporation of water through the tight junction portion can be prevented. In contrast, when the expression level of the OR6M1 or OR6V1 gene in a specimen is low, the skin barrier function is poor so that the epidermal cell differentiation is diminished, the skin moisturization is diminished, the tight junction is loosened, thus the evaporation of water is not effectively prevented, and the epidermal cell tissue is loosened.

Consequently, when the expression level of the OR6M1 or OR6V1 gene in a specimen is increased as compared to that of a normal control group of an undamaged epidermis, it can be diagnosed that the skin barrier function is excellent, the epidermal cell differentiation is promoted, the skin moisturization is enhanced, the tight junction is improved, the epidermal cells tightly bind to each other, thus the epidermal cell tissue is solid.

The test of the expression level of the OR6M1 or OR6V1 gene can be conducted by conventional methods known in the art. For example, the test may comprise measuring the level of at least one of the mRNA of the OR6M1 or OR6V1 gene in a specimen or the protein encoded by the OR6M1 or OR6V1 gene.

In a specific embodiment, the measurement of the mRNA level of the OR6M1 or OR6V1 gene can be performed using polynucleotides which specifically bind to the whole or a part of the mRNA, but it is not limited thereto.

For example, the measurement of the mRNA level of the OR6M1 or OR6V1 gene can be performed using a probe for mRNA test of OR6M1 or OR6V1 gene or a primer pair capable of amplifying mRNA of the OR6M1 or OR6V1 gene.

In the case of using the probe, the measurement of the mRNA level of the OR6M1 or OR6V1 gene can be performed by measuring the amount of the transcript hybridized with the probe in the specimen.

The probe is a polynucleotide which specifically binds to at least one gene of OR6M1 or OR6V1 and may be a polynucleotide of the gene or a fragment comprising at least 10 consecutive nucleotides of the gene or a polynucleotide complementary to a polynucleotide of the gene or a polynucleotide complementary to a fragment comprising at least 10 consecutive nucleotides of the gene.

In the case of using the primer pair, the measurement of the mRNA level of the OR6M1 or OR6V1 gene can be performed by measuring the amount of the polynucleotide amplified using a primer pair capable of amplifying at least one mRNA of the OR6M1 or OR6V1 gene.

The primer pair may include 10 to 20 consecutive nucleotide fragments of a polynucleotide of the gene and 10 to 20 consecutive nucleotide fragments complementary to a polynucleotide of the gene.

In another embodiment, the measurement of the level of a protein encoded by at least one of the OR6M1 or OR6V1 gene can be performed using an antibody which specifically binds to the protein.

In the case of using the antibody the measurement of the level of a protein encoded by the OR6M1 or OR6V1 gene can be performed by measuring the amount of a protein hybridized with the antibody.

The antibody may be a monoclonal antibody of a protein encoded by at least one gene of OR6M1 or OR6V1.

For the test of the expression level of at least one gene of OR6M1 or OR6V1, polymerase chain reaction (PCR), reverse transcriptase polymerase chain reaction (RT-PCR), northern blot analysis, western blot analysis, dot blot assay, an enzyme-linked immunosorbent assay (ELISA), and a microarray, for example, a TaqMan (TM) probe method using an allele-specific hybridization, a probe method using allele-specific hybridization through a polymerization reaction, a restriction fragment length polymorphism (RFLP) method using restriction enzymes, a dynamic allele-specific hybridization (DASH) method using a melting temperature (Tm), pyrosequencing using polymerization, a microarray method using allele-specific extension, and the like may be utilized, but the method is not limited thereto.

In a specific embodiment, the expression level of at least one gene of OR6M1 or OR6V1 may be 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.1 times or more, 2.2 times or more, 2.3 times or more, 2.4 times or more, 2.5 times or more, 2.6 times or more, 2.7 times or more, 2.8 times or more, 2.9 times or more, 3.0 times or more, 3.1 times or more, 3.2 times or more, 3.3 times or more, 3.4 times or more, 3.5 times or more, 3.6 times or more, 3.7 times or more, 3.8 times or more, 3.9 times or more, 4.0 times or more, 4.1 times or more, 4.2 times or more, 4.3 times or more, 4.4 times or more, 4.5 times or more, 4.6 times or more, 4.7 times or more, 4.8 times or more, 4.9 times or more, or 5.0 times or more and 20.0 times or less, 19.5 times or less, 19.0 times or less, 18.5 times or less, 18.0 times or less, 17.5 times or less, 17.0 times or less, 16.5 times or less, 16.0 times or less, 15.5 times or less, 15.0 times or less, 14.5 times or less, 14.0 times or less, 13.5 times or less, 13.0 times or less, 12.5 times or less, 12.0 times or less, 11.5 times or less, 11.0 times or less, 10.5 times or less, 10.0 times or less, 9.5 times or less, 9.0 times or less, 9.0 times or less, 8.5 times or less, 8.0 times or less, 7.5 times or less, 7.0 times or less, 6.5 times or less, 6.0 times or less, 5.5 times or less, or 5.0 times or less the normal value (expression level of normal control group). It may be diagnosed that the skin barrier function is excellent when the expression level of at least one gene of OR6M1 or OR6V1 is, for example, from 1.1 to 20.0 times, for example, from 1.5 to 10.0 times, and for example, from 2 times to 8 times the normal value.

According to an embodiment of the present description it is possible to provide a biomarker composition for diagnosis of skin barrier function, with which the expression level of at least one gene of OR6M1 or OR6V1 is tested. By using the composition of the present embodiment, the expression level of at least one gene of OR6M1 or OR6V1 in a specimen can be tested to diagnose the skin barrier function.

The biomarker composition may contain at least one of a probe or primer pair which specifically binds to mRNA or an antibody which specifically binds to a protein encoded by at least one gene of OR6M1 or OR6V1.

The specimen, the gene, the probe, the primer pair, and the antibody are the same as those described above with regard to the diagnostic method according to an embodiment of the present description, and thus the description thereof will be omitted.

According to an embodiment of the present description, it is possible to provide a kit for diagnosis of skin barrier function, with which the expression level of at least one gene of OR6M1 or OR6V1 is tested. By using the kit of the present embodiment, the expression level of at least one gene of OR6M1 or OR6V1 in a specimen can be tested to diagnose the skin barrier function.

The kit may comprise the composition according to an embodiment of the present description described above. The composition is the same as that described above and thus the description thereof will be omitted.

In a specific embodiment, the kit comprises at least one polynucleotide which specifically binds to at least one gene of OR6M1 and OR6V1; and a solid support in which the at least one polynucleotide is bound to the surface, and
the polynucleotide which specifically binds to the at least one gene of OR6M1 and OR6V1 may comprise a polynucleotide of the gene or a fragment containing at least 10 consecutive nucleotides of the gene, or a polynucleotide complementary to a polynucleotide of the gene or a polynucleotide complementary to a fragment containing at least 10 consecutive nucleotides of the gene.

In another specific embodiment, the kit may comprise a primer pair capable of amplifying mRNA of the polynucleotide of at least one gene of OR6M1 and OR6V1.

In still another embodiment, the kit may comprise a monoclonal antibody to a protein encoded from at least one gene of OR6M1 and OR6V1.

According to an embodiment of the present description, it is possible to provide a method for screening a skin barrier function improving substance through a change in the expression level of at least one gene of OR6M1 and OR6V1 by a test substance.

The screening method may comprise treating an epidermal cell with a test substance; and confirming relative expression levels of at least one gene of OR6M1 and OR6V1 before and after the treatment with a test substance in the epidermal cell treated with a test substance.

It may be judged that a test substance is a skin barrier function improving substance when the relative expression levels of the genes before and after the treatment with a test substance are compared and the relative expression level of the gene after the treatment with a test substance is higher than that before the treatment with a test substance.

For example, the relative expression level of the gene may be 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.1 times or more, 2.2 times or more, 2.3 times or more, 2.4 times or more, 2.5 times or more, 2.6 times or more, 2.7 times or more, 2.8 times or more, 2.9 times or more, 3.0 times or more, 3.1 times or more, 3.2 times or more, 3.3 times or more, 3.4 times or more, 3.5 times or more, 3.6 times or more, 3.7 times or more, 3.8 times or more, 3.9 times or more, 4.0 times or more, 4.1 times or more, 4.2 times or more, 4.3 times or more, 4.4 times or more, 4.5 times or more, 4.6 times or more, 4.7 times or more, 4.8 times or more, 4.9 times or more, or 5.0 times or more and 20.0 times or less, 19.5 times or less, 19.0 times or less, 18.5 times or less, 18.0 times or less, 17.5 times or less, 17.0 times or less, 16.5 times or less, 16.0 times or less, 15.5 times or less, 15.0 times or less, 14.5 times or less, 14.0 times or less, 13.5 times or less, 13.0 times or less, 12.5 times or less, 12.0 times or less, 11.5 times or less, 11.0 times or less, 10.5 times or less, 10.0 times or less, 9.5 times or less, 9.0 times or less, 9.0 times or less, 8.5 times or less, 8.0 times or less, 7.5 times or less, 7.0 times or less, 6.5 times or less, 6.0 times or less, 5.5 times or less, or 5.0 times or less that before the treatment with a test substance. It may be judged that a test substance is a skin barrier function improving substance when the relative expression level is, for example, from 1.1 to 20.0 times, for example, from 1.5 to 10.0 times, and for example, from 2 times to 8 times that before the treatment with a test substance.

### [Embodiments]

Hereinafter, the present invention will be described in detail with reference to Examples, Comparative Examples and Test Examples. It will be apparent to those skilled in the art that these are merely illustrative examples of the present invention in order to more specifically explain the present invention and the scope of the present invention is not limited by these Examples, Comparative Examples and Test Examples.

### [Test Example 1] Confirmation of expression pattern of olfactory receptor gene in epidermal cell

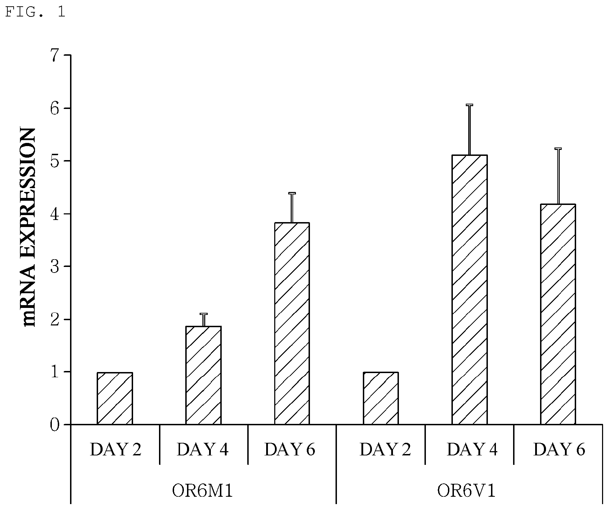

The neonatal keratinocyte (normal human epidermal keratinocyte: P988, LONZA) was inoculated into 60 mm cell culture dishes using keratinocyte growth medium (KGM) at a density of 1.25 × 10⁴ cells/dish and then cultured in a 5% CO₂ incubator at 37°C until to have a confluency of 80%. The change in expression of the olfactory receptor depending on the number of days elapsed was confirmed while culturing the cells for 6 days thereafter. The results are illustrated in FIG. 1. The change in expression was determined by removing the medium for cell growth, adding 1 ml of Trizol (Invitrogen) to the cells, isolating RNA according to the RNA isolation method (Invitrogen), quantifying RNA at 260 nm using an ultraviolet spectrophotometer (HEWLETT PACKARD), and conducting reverse transcription-polymerase chain reaction (RT-PCR). For the gene analysis of OR6M1 and OR6V1 in each sample, a TaqMan probe (Hs02339286_s1, Hs01073030_s1) was used and correction was conducted based on the complementary gene RPL13A.

From the results of FIG. 1, the expression of OR6M1 and OR6V1 genes increases as the number of days for which the cells are cultured increases, the expression level of the gene increases until the sixth day, and thus the gene has been confirmed to be a gene of which the expression increases as epidermal cells differentiate.

### [Test Example 2] Expression of olfactory receptor gene in epidermal cell depending on irradiation with ultraviolet light

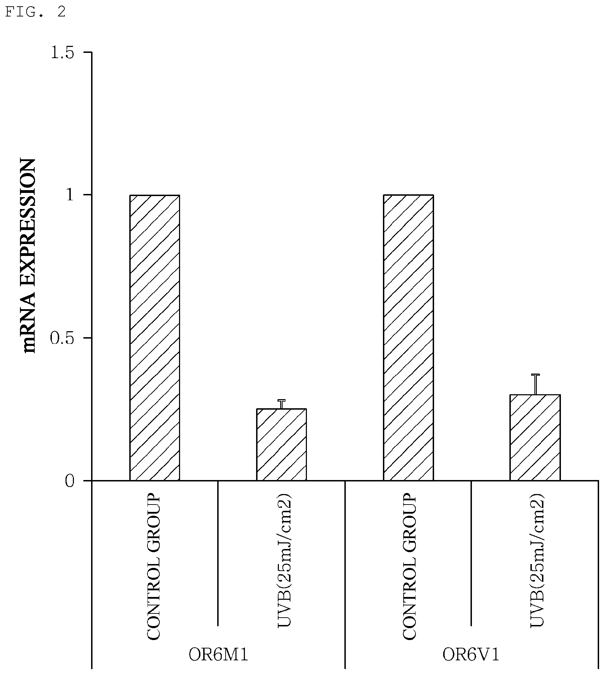

The neonatal keratinocyte (normal human epidermal keratinocyte: P988, LONZA) was inoculated into 60 mm cell culture dishes using keratinocyte growth medium (KGM) at a density of 1.25 × 10⁴ cells/dish and then cultured in a 5% CO₂ incubator at 37°C until to have a confluency of 80%. The cultured cells were treated with ultraviolet ray (UVB: 25 mJ/cm²) and cultured for 2 days, and then the change in expression was confirmed. The results are illustrated in FIG. 2. The change in expression was measured in the same manner as in Test Example 1 above. A group which was not treated with ultraviolet light was used as the control group.

From the results of FIG. 2, it can be confirmed that the expression levels of OR6M1 and OR6V1 genes are remarkably decreased by irradiation with ultraviolet light.

### [Test Example 3] Knock-down of olfactory receptor (OR6M1 and OR6V1) gene

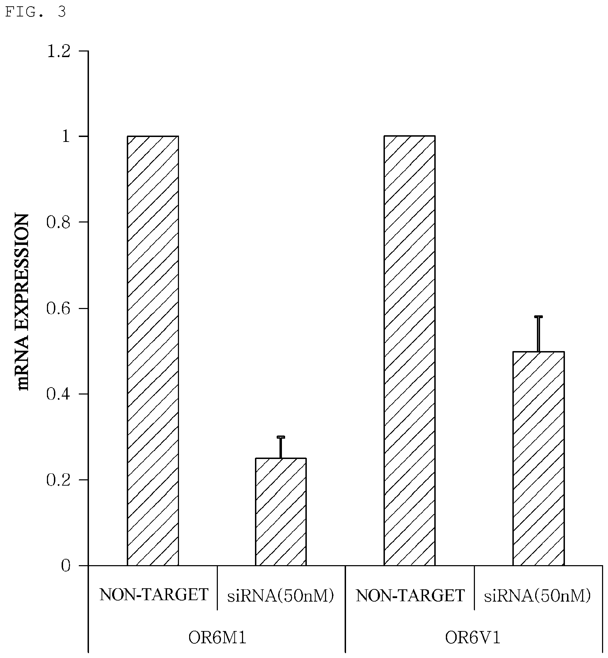

In order to confirm the gene function of OR6M1 and OR6V1 in epidermal cells, cells were cultured in the same manner as in Example 1 and then transfected into keratinocytes using siRNA (50 nM, purchased from Dharmacon, trade name ON-TARGET plus siRNA Reagents) and lipofectamine for each of OR6M1 and OR6V1 genes to selectively knock down OR6M1 and OR6V1, and then the degree of knock-down of the two genes was compared to that of a non-target after 2 days. The results are illustrated in FIG. 3.

From the results of FIG. 3, it can be confirmed that the expression levels of OR6M1 and OR6V1 genes all decrease to 50% or less.

### [Test Example 4] Influence of function loss of olfactory receptor (OR6M1 and OR6V1) genes on epidermal differentiation

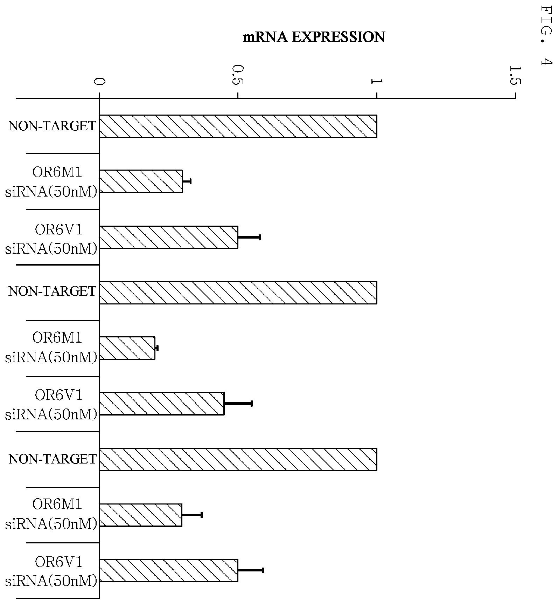

The expression levels of keratin 1 (KRT1, Hs01549614_g1), keratin 10 (KRT10, Hs01043114_g1) and loricrin (LOR, Hs01894962_s1) genes, which are epidermal differentiation markers, in keratinocytes in which OR6M1 and OR6V1 genes were respectively knocked down in the same manner as in Test Example 3 were compared to those in a non-target in which OR6M1 and OR6V1 genes were not knocked down. The expression levels of these gene were confirmed in the same manner as in Test Example 1 using Real-Time RT-PCR (RT-PCR). The results are illustrated in FIG. 4.

From the results of FIG. 4, it can be confirmed that the expression of keratin 1, keratin 10 and loricrin genes remarkably decreases as the expression of OR6M1 and OR6V1 genes are inhibited, and it can be thus seen that the function of OR6M1 and OR6V1 greatly affects the epidermal differentiation.

### [Test Example 5] Influence of function loss of olfactory receptor (OR6M1 and OR6V1) gene on stratum corneum moisturizing factor

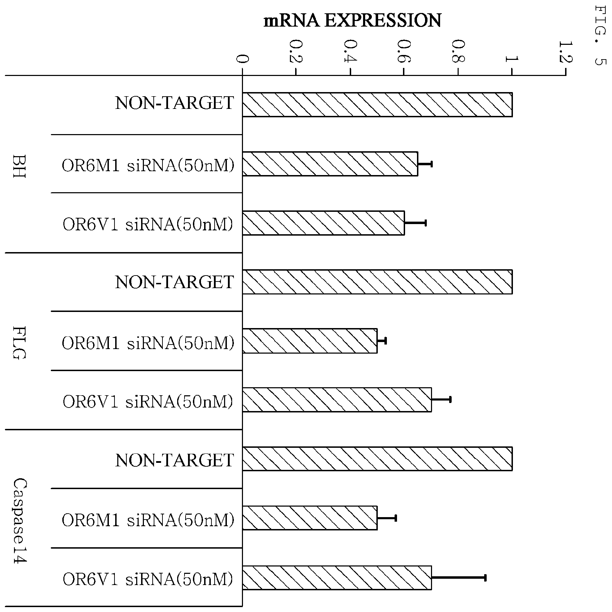

The expression levels of bleomycin hydrolase (BLMH; BH, Hs00166071_m1), filaggrin (FLG, Hs00856927_g1) and caspase 14 (CSAP14, Hs00201637_m1) genes, which are water holding metabolism factors of stratum corneum, in the keratinocytes in which OR6M1 and OR6V1 genes were respectively knocked down in the same manner as in Test Example 3 were compared to those in a non-target in which OR6M1 and OR6V1 genes were not knocked down. The expression levels of these gene were confirmed in the same manner as in Test Example 1 using Real-Time RT-PCR (RT-PCR). The results are illustrated in FIG. 5.

From the results of FIG. 5, it can be confirmed that the expression of bleomycin hydrolase (BH), filaggrin (FLG) and caspase 14 genes decreases to 50% or less as the expression of OR6M1 and OR6V1 genes are inhibited, and it can be thus seen that a decrease in function of OR6M1 and OR6V1 leads to deformation of the outermost stratum corneum and a decrease in moisturizing metabolic function is thus caused.

### [Test Example 6] Change in tight junction due to function loss of olfactory receptor (OR6M1 and OR6V1) gene

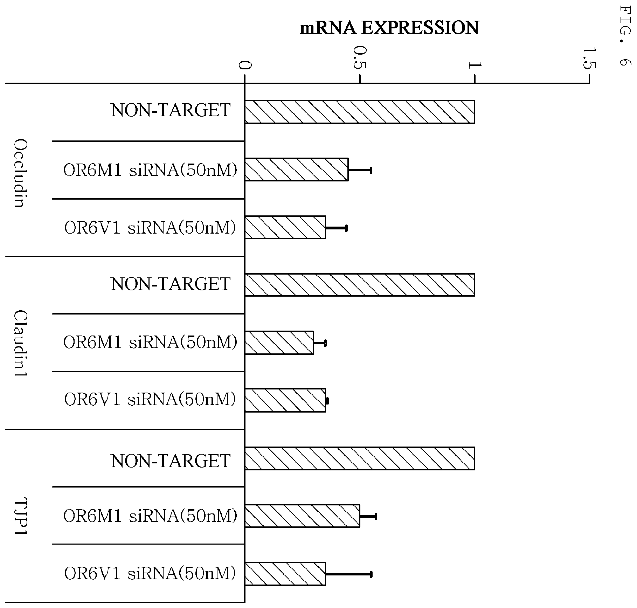

The expression levels of occuldin (OCLN, Hs00170162_m1), claudin1 (CLDN1, Hs00221623_m1) and TJP1 (Hs01551861_m1) genes, which are factors related to tight junction, in keratinocytes in which OR6M1 and OR6V1 genes were respectively knocked down in the same manner as in Test Example 3 were compared to those in a non-target in which OR6M1 and OR6V1 genes were not knocked down. The expression levels of these gene were confirmed in the same manner as in Test Example 1 using Real-Time RT-PCR (RT-PCR). The results are illustrated in FIG. 6.

From the results of FIG. 6, it can be confirmed that the expression of occuldin, claudin1, and TJP1 genes decreases to 50% or less as the expression of OR6M1 and OR6V1 genes are inhibited, and it can be thus seen that a decrease in function of OR6M1 and OR6V1 leads to a change in the intercellular adhesion in the granular layer and stratum spinosum below the stratum corneum.

## Claims

1. Use of a composition comprising a reagent for detecting an expression level of at least one gene of OR6M1 and OR6V1 in a method for diagnosing a skin barrier function, the skin barrier function comprises at least one of epidermal cell differentiation, skin moisturization, and tight junction of epidermal cells.

2. The use according to claim 1, wherein the reagent comprises at least one of a probe or primer pair which specifically binds to mRNA of at least one gene of OR6M1 and OR6V1; or an antibody which specifically binds to a protein encoded by at least one gene of OR6M1 or OR6V1.

3. The use according to claim 2, wherein the probe comprises a polynucleotide of the gene or a fragment comprising at least 10 consecutive nucleotides of the gene, or a polynucleotide complementary to a polynucleotide of the gene or a polynucleotide complementary to a fragment comprising at least 10 consecutive nucleotides of the gene.

4. The use according to claim 2, wherein the primer pair includes 10 to 20 consecutive nucleotide fragments of a polynucleotide of the gene and 10 to 20 consecutive nucleotide fragments complementary to a polynucleotide of the gene.

5. The use according to claim 2, wherein the antibody is a monoclonal antibody to a protein encoded by at least one gene of OR6M1 and OR6V1.

6. The use according to any one of claims 1-5, wherein it is diagnosed that skin barrier function is excellent when a gene expression level is increased as compared to a normal value, optionally, wherein it is diagnosed that skin barrier function is damaged when a gene expression level is decreased as compared to a normal value.

7. An ex vivo method for screening a skin barrier function improving substance, comprising:
- treating an epidermal cell isolated from human with a test substance;
and
- confirming relative expression levels of at least one gene of OR6M1 and OR6V1 before and after the treatment with the test substance in the epidermal cell treated with the test substance,
wherein improvement of the skin barrier function includes at least one of promotion of epidermal cell differentiation, skin moisturization, and improvement of tight junction of epidermal cells.

8. The method for screening according to claim 7, further comprising judging the test substance as a skin barrier function improving substance when a relative expression level of the gene in the epidermal cell treated with a test substance is higher as compared to a relative expression level before the treatment with a test substance.

9. The method for screening according to claim 8, wherein the test substance is judged as a skin barrier function improving substance when a relative expression level of the gene is 1.1 times or more the relative expression level before the treatment with a test substance.

10. The method for screening according to any one of claims 7-9, wherein the epidermal cell is at least one of a keratinocyte or a melanocyte.

## Patentansprüche

1. Verwendung einer Zusammensetzung, die ein Reagenz zum Nachweis eines Expressionsniveaus mindestens eines Gens von OR6M1 und OR6V1 umfasst, in einem Verfahren zur Diagnose einer Hautbarrierefunktion, wobei die Hautbarrierefunktion mindestens eines von epidermaler Zelldifferenzierung, Hautbefeuchtung und Schlussleiste von epidermalen Zellen umfasst.

2. Verwendung nach Anspruch 1, wobei das Reagenz mindestens eine Sonde oder ein Primerpaar umfasst, das spezifisch an mRNA von mindestens einem Gen von OR6M1 und OR6V1 bindet; oder einen Antikörper, der spezifisch an ein Protein bindet, das von mindestens einem Gen von OR6M1 oder OR6V1 kodiert wird.

3. Verwendung nach Anspruch 2, wobei die Sonde ein Polynukleotid des Gens oder ein Fragment, das mindestens 10 aufeinanderfolgende Nukleotide des Gens umfasst, oder ein Polynukleotid, das komplementär zu einem Polynukleotid des Gens oder einem Polynukleotid, das komplementär zu einem Fragment, das mindestens 10 aufeinanderfolgende Nukleotide des Gens umfasst, ist, umfasst.

4. Verwendung nach Anspruch 2, wobei das Primerpaar 10 bis 20 aufeinanderfolgende Nukleotidfragmente eines Polynukleotids des Gens und 10 bis 20 aufeinanderfolgende Nukleotidfragmente komplementär zu einem Polynukleotid des Gens umfasst.

5. Verwendung nach Anspruch 2, wobei der Antikörper ein monoklonaler Antikörper gegen ein Protein ist, das von mindestens einem Gen von OR6M1 und OR6V1 kodiert wird.

6. Verwendung nach einem der Ansprüche 1-5, wobei diagnostiziert wird, dass die Hautbarrierefunktion ausgezeichnet ist, wenn ein Genexpressionsniveau im Vergleich zu einem Normalwert erhöht ist,
optional, wobei diagnostiziert wird, dass die Hautbarrierefunktion beschädigt ist, wenn ein Genexpressionsniveau im Vergleich zu einem normalen Wert verringert ist.

7. Ex-vivo-Verfahren zum Screening einer die Hautbarrierefunktion verbessernden Substanz, umfassend:
- Behandeln einer vom Menschen isolierten Epidermiszelle mit einer Testsubstanz; und
- Bestätigen der relativen Expressionsniveaus von mindestens einem Gen von OR6M1 und OR6V1 vor und nach der Behandlung mit der Testsubstanz in der mit der Testsubstanz behandelten Epidermiszelle,
wobei die Verbesserung der Hautbarrierefunktion mindestens eines aus der Förderung der epidermalen Zelldifferenzierung, der Befeuchtung der Haut und der Verbesserung der Schlussleiste der epidermalen Zellen einschließt.

8. Screening-Verfahren nach Anspruch 7, ferner umfassend das Beurteilen der Testsubstanz als eine die Hautbarrierefunktion verbessernde Substanz, wenn ein relatives Expressionsniveau des Gens in der mit einer Testsubstanz behandelten epidermalen Zelle höher ist im Vergleich zu einem relativen Expressionsniveau vor der Behandlung mit einer Testsubstanz.

9. Screening-Verfahren nach Anspruch 8, wobei die Testsubstanz als eine die Hautbarrierefunktion verbessernde Substanz beurteilt wird, wenn ein relatives Expressionsniveau des Gens das 1,1-fache oder mehr des relativen Expressionsniveaus vor der Behandlung mit einer Testsubstanz beträgt.

10. Screening-Verfahren nach einem der Ansprüche 7 bis 9, wobei die epidermale Zelle mindestens eine von einem Keratinozyten oder einem Melanozyten ist.

## Revendications

1. Utilisation d'une composition comprenant un réactif pour détecter un niveau d'expression d'au moins un gène de OR6M1 et OR6V1 dans une méthode de diagnostic d'une fonction de barrière cutanée, la fonction de barrière cutanée comprenant au moins l'une parmi la différenciation des cellules épidermiques, l'hydratation de la peau et la jonction serrée des cellules épidermiques.

2. Utilisation selon la revendication 1, dans laquelle le réactif comprend au moins un élément parmi une sonde ou une paire d'amorces qui se lie spécifiquement à l'ARNm d'au moins un gène de OR6M1 et OR6V1 ; ou un anticorps qui se lie spécifiquement à une protéine codée par au moins un gène de OR6M1 ou OR6V1.

3. Utilisation selon la revendication 2, dans laquelle la sonde comprend un polynucléotide du gène ou un fragment comprenant au moins 10 nucléotides consécutifs du gène, ou un polynucléotide complémentaire d'un polynucléotide du gène ou un polynucléotide complémentaire d'un fragment comprenant au moins 10 nucléotides consécutifs du gène.

4. Utilisation selon la revendication 2, dans laquelle la paire d'amorces comprend 10 à 20 fragments nucléotidiques consécutifs d'un polynucléotide du gène et 10 à 20 fragments nucléotidiques consécutifs complémentaires d'un polynucléotide du gène.

5. Utilisation selon la revendication 2, dans laquelle l'anticorps est un anticorps monoclonal dirigé contre une protéine codée par au moins un gène parmi OR6M1 et OR6V1.

6. Utilisation selon l'une quelconque des revendications 1 à 5, dans laquelle il est diagnostiqué que la fonction de barrière cutanée est excellente lorsqu'un niveau d'expression génique est augmenté par rapport à une valeur normale,
optionnellement, dans laquelle il est diagnostiqué que la fonction de barrière cutanée est endommagée lorsqu'un niveau d'expression de gène est diminué par rapport à une valeur normale.

7. Procédé ex vivo de criblage d'une substance améliorant la fonction de barrière cutanée, comprenant :
- le traitement d'une cellule épidermique isolée d'un humain avec une substance d'essai ; et
- la confirmation des niveaux d'expression relatifs d'au moins un gène de OR6M1 et OR6V1 avant et après le traitement avec la substance testée dans la cellule épidermique traitée avec la substance testée,
dans lequel l'amélioration de la fonction de barrière cutanée comprend au moins l'un des éléments suivants : promotion de la différenciation des cellules épidermiques, hydratation de la peau et amélioration de la jonction serrée des cellules épidermiques.

8. Procédé de criblage selon la revendication 7, comprenant en outre le fait de juger la substance d'essai comme une substance améliorant la fonction de barrière cutanée lorsqu'un niveau d'expression relative du gène dans la cellule épidermique traitée avec une substance d'essai est plus élevé par rapport à un niveau d'expression relative avant le traitement avec une substance d'essai.

9. Procédé de criblage selon la revendication 8, dans lequel la substance d'essai est jugée comme une substance améliorant la fonction de barrière cutanée lorsqu'un niveau d'expression relative du gène est 1,1 fois ou plus le niveau d'expression relative avant le traitement avec une substance d'essai.

10. Procédé de criblage selon l'une quelconque des revendications 7 à 9, dans lequel la cellule épidermique est au moins l'un parmi un kératinocyte ou un mélanocyte.
